(19) Europäisches Patentamt
European Patent Office
Office européen des brevets

(11) Veröffentlichungsnummer: **0 400 292**
**A2**

(12) **EUROPÄISCHE PATENTANMELDUNG**

(21) Anmeldenummer: 90105639.0

(22) Anmeldetag: 24.03.90

(51) Int. Cl.⁵: **C07C 67/38, C07C 69/74**

(30) Priorität: 27.05.89 DE 3917329

(43) Veröffentlichungstag der Anmeldung:
05.12.90 Patentblatt 90/49

(84) Benannte Vertragsstaaten:
**DE FR GB NL**

(71) Anmelder: **HÜLS AKTIENGESELLSCHAFT**
**Patentabteilung / PB 15 · Postfach 13 20**
**D-4370 Marl 1(DE)**

(72) Erfinder: **Keil, Thomas, Dr.**
**Siegerlandstrasse 27**
**D-4350 Recklinghausen(DE)**

(54) Verfahren zur Herstellung von Cyclooctandicarbonsäurediestern.

(57)

2.1 Cyclooctandicarbonsäurediester können durch Hydrocarboxyalkylierung von 1,5-Cyclooctadien hergestellt werden. Dazu sind bisher Palladiumkatalysatoren erforderlich.

2.2 Erfindungsgemäß können Cycloctandicarbonsäurediester nun durch Hydrocarboxyalkylierung von 1,5-Cyclooctadien an Kobaltkatalysatoren in Gegenwart tertiärer Amine hergestellt werden.

2.3 Herstellung von Cyclooctandicarbonsäurediestern

EP 0 400 292 A2

## Verfahren zur Herstellung von Cyclooctandicarbonsäurediestern

Die Erfindung betrifft ein Verfahren zur Herstellung von Cyclooctandicarbonsäurediestern durch Hydrocarboxyalkylierung von 1,5-Cyclooctadien.

Cyclooctandicarbonsäurediester sind wertvolle Zwischenprodukte zur Darstellung von Alkydharzen, Polyamiden, Polyestern oder Schmiermitteln.

Nach J. Falbe, Synthesen mit Kohlenmonoxid, 1967, Seite 102 ff., kann man Cyclooctandicarbonsäurediester durch Hydrocarboxyalkylierung von 1,5-Cyclooctadien an Palladiumkatalysatoren herstellen. Die gleiche Reaktion soll an Kobaltkontakten nur zu gesättigten Cyclooctancarbonsäureestern führen.

Auch nach BE 613 730 wird bei der zur Hydrocarboxyalkylierung analogen Hydrocarboxylierung von 1,5-Cyclooctadien mit Kohlenmonoxid und Wasser an Nickelkatalysatoren nur die gesättigte Cyclooctancarbonsäure synthetisiert.

Bisher können Cyclooctandicarbonsäurediester nur mit Hilfe von Palladiumkatalysatoren aus 1,5-Cyclooctadien hergestellt werden.

Aufgabe der vorliegenden Erfindung ist es, ein Verfahren bereitzustellen, bei dem die Herstellung von Cyclooctandicarbonsäurediestern aus 1,5-Cyclooctadien durch eine Verbindung eines unedlen Metalls katalysiert wird.

Überraschenderweise wird die Aufgabe dadurch gelöst, daß man die Hydrocarboxyalkylierung in Gegenwart einer Kobaltverbindung und eines tertiären Amins bei 100 bis 200 °C und 150 bis 350 bar durchführt.

Eine geeignete Kobaltverbindung ist Kobalttetracarbonylwasserstoff $HCo(CO)_4$. Es können aber auch Kobaltsalze, wie Kobalt-(II)-acetat, -naphthenat, -stearat, -carbonat oder -chlorid, Kobaltoxide oder Kobaltkomplexe, wie Dikobaltoctacarbonyl, eingesetzt werden. Diese Kobaltverbindungen erfordern in der Anfangsphase der Hydrocarboxyalkylierung neben Kohlenmonoxid 0,1 bis 10 Mol-% Wasserstoff, bezogen auf Mole Kohlenmonoxid. Dabei wird unter den gegebenen Reaktionsbedingungen von 100 bis 200 °C und 150 bis 350 bar Kobalttetracarbonylwasserstoff gebildet, das vermutlich die eigentliche katalytisch aktive Verbindung ist. Die Bildung des Kobalttetracarbonylwasserstoffs ist im allgemeinen nach einer halben Stunde beendet.

Auch beim Einsatz von Kobalttetracarbonylwasserstoff ist ein kleiner Anfangsgehalt an Wasserstoff hilfreich, da Wasserstoff verbrauchten Katalysator regeneriert.

Bezogen auf eingesetztes 1,5-Cyclooctadien verwendet man vorzugsweise 0,5 bis 5 Mol-% Kobaltverbindung.

Als tertiäres Amin, das als Promotor wirkt, kommen Pyridin und nicht-ortho-alkylierte Pyridine, wie z. B. 3- und 4-Picolin, 3,4- und 3,5-Lutidin und 3- und 4-Ethylpyridin, infrage. Vorzugsweise wird 4-Picolin eingesetzt. Das Molverhältnis tertiäres Amin : Kobaltverbindung beträgt vorzugsweise 2 : 1 bis 10 : 1.

Als Lösemittel und Reagenz verwendet man im allgemeinen Alkohole mit 1 bis 6 C-Atomen, wie Methanol, Ethanol, Isopropanol, Butanol oder Hexanol. Methanol ist dabei bevorzugt. Vorzugsweise stellt man ein Molverhältnis 1,5-Cyclooctadien : Alkohol von 1 : 2 bis 1 : 4 ein.

Innerhalb des genannten Temperaturbereichs wird vorzugsweise zuerst eine niedrige und dann eine hohe Temperatur eingestellt. In der ersten Stufe liegt die Temperatur dann bei 120 bis 150 °C und in der zweiten Stufe bei 150 bis 200 °C.

Bei der Hydrocarboxyalkylierung wird ein Druck von 200 bis 300 bar bevorzugt.

Die Reaktion wird im allgemeinen in einem Autoklaven durchgeführt.

Die Gesamtreaktionszeit beträgt meist 24 bis 48 Stunden. Aus dem Reaktionsgemisch können die Cyclooctandicarbonsäurediester nach üblichen Methoden isoliert werden. Es werden immer Isomerengemische erhalten.

Das erfindungsgemäße Verfahren benötigt keine Edelmetallkatalysatoren. Preisgünstigere Katalysatoren können eingesetzt werden.

Das Verfahren liefert die Cyclooctandicarbonsäurediester in Ausbeuten von meist über 50 Gewichtsprozent. Die störenden gesättigten Cyclooctancarbonsäureester werden dagegen in Mengen von nur 10 bis 20 % erhalten. Als weiteres wichtiges Nebenprodukt werden die entsprechenden Cyclooctencarbonsäureester gebildet, die nach Abtrennung des Hauptprodukts erneut einer Hydrocarboxyalkylierung unterworfen werden können. Dadurch kann die Ausbeute an gewünschtem Produkt noch gesteigert werden.

Beispiele

In den Beispielen, die die Erfindung verdeutlichen sollen, wird ein 5-1-VA-Stahlautoklav verwendet. Die Reaktionskomponenten 1,5-Cyclooctadien, Alkohol, Kobalt-(II)-salz und 4-Picolin werden eingefüllt und auf Reaktionstemperatur gebracht. Dann wird Wasserstoff aufgedrückt, worauf durch Aufdrücken von Kohlenmonoxid der Gesamtdruck aufgebaut wird.

Der Druckabfall während der Reaktion wird durch ständige Kohlenmonoxid-Zufuhr ausgeglichen. Nach beendeter Reaktion wird auf Raumtemperatur abgekühlt und entspannt. Die gaschromatographisch bestimmten Ausbeuten werden in Mol-%, bezogen auf eingesetztes 1,5-Cyclooctadien, angegeben.

| Beispiel 1 | |
|---|---|
| Einwaage: | |
| 951,5 g | (8,8 Mol) 1,5-Cyclooctadien |
| 592,1 g | (18,5 Mol) Methanol |
| 98,3 g | (1,06 Mol) 4-Picolin |
| 155,6 g | (0,26 Mol) Co-(II)-naphthenat (10%ig an Co) |
| Reaktionsbedingungen: | |
| $P_{H_2}$ = 10 bar (anfangs), P ges = 280 bar<br>1. Stufe: 140 °C/24 h<br>2. Stufe: 190 °C/12 h<br>Man erhält: | |
| 4,5 % | Bicyclo[3.3.1]non-2-en-9-on |
| 10 % | Cyclooctencarbonsäuremethylester |
| 17 % | Cyclooctencarbonsäuremethylester |
| 47 % | Cyclooctandicarbonsäuredimethylester |
| ‾78,5 % | |

Der Rest enthält isomerisiertes Ausgangsprodukt und Hochsieder. Bei einer fraktionierten Destillation geht bei 110 bis 120 °C und 0,9 mbar der Diester (Isomerengemisch) über. Das Produkt wird gaschromatographisch und durch Massenspektrum identifiziert.

| Beispiel 2 | |
|---|---|
| Einwaage: | |
| 951,5 g<br>592,1 g<br>98,3 g<br>155,6 g | 1,5-Cyclooctadien<br>Methanol<br>4-Picolin<br>Co-(II)-naphthenat (10%ig an Co) |
| Rea-<br>ktio-<br>nsb-<br>edin-<br>gun-<br>gen: | |
| $P_{H2}$ = 10 bar (anfangs), $P_{ges}$ = 280 bar<br>1. Stufe: 140 ° C 24 h<br>2. Stufe: 170 ° C 12 h<br>Man erhält: | |
| 5,3 %<br>13 %<br>13,4 %<br>54 %<br>85,7 % | Bicyclo[3.3.1]non-2-en-9-on<br>Cyclooctencarbonsäuremethylester<br>Cyclooctencarbonsäuremethylester<br>Cyclooctandicarbonsäuredimethylester |

| Beispiel 3 | |
|---|---|
| Einwaage: | |
| 951,5 g<br>592,1 g<br>98,3 g<br>155,6 g | 1,5-Cyclooctadien<br>Methanol<br>4-Picolin<br>Co-(II)-naphthenat (10%ig an Co) |
| Reaktionsbedingungen: | |
| $P_{H2}$ = 10 bar (anfangs), $P_{ges}$ = 280 bar<br>1. Stufe: 140 ° C 24 h<br>2. Stufe: 160 ° C 12 h<br>Man erhält: | |
| 5,3 %<br>13,5 %<br>12,3 %<br>54 %<br>85,1 % | Bicyclo[3.3.1]non-2-en-9-on<br>Cyclooctencarbonsäuremethylester<br>Cyclooctancarbonsäuremethylester<br>Cyclooctandicarbonsäuredimethylester |

| Beispiel 4 | |
|---|---|
| Einwaage: | |
| 1 005,5 g<br>625,7 g<br>103,9 g<br>69,5 g | 1,5-Cyclooctadien<br>Methanol<br>4-Picolin<br>Co-(II)-acetat • $4H_2O$ |
| Reaktionsbedingungen: | |
| $P_{H2}$ = 10 bar (anfangs), $P_{ges}$ = 270 bar<br>1. Stufe: 140 °C/24 h<br>2. Stufe: 160 °C/12 h<br>Man erhält: | |
| 6,2 %<br>12,1 %<br>12,4 %<br>54,8 %<br>85,5 % | Bicyclo[3.3.1]non-2-en-9-on<br>Cyclooctencarbonsäuremethylester<br>Cyclooctancarbonsäuremethylester<br>Cyclooctandicarbonsäuredimethylester |

| Beispiel 5 | |
|---|---|
| Ei-<br>nw-<br>aa-<br>ge: | |
| 832,5 g<br>744,9 g<br>86,0 g<br>136,1 g | 1,5-Cyclooctadien<br>Ethanol<br>4-Picolin<br>Co-(II)-naphthenat (10%ig an Co) |
| Reaktionsbedingungen: | |
| $P_{H2}$ = 10 bar (anfangs), $P_{ges}$ = 280 bar<br>1. Stufe: 140 °C/24 h<br>2. Stufe: 160 °C/12 h<br>Man erhält: | |
| 5,9 %<br>11,7 %<br>15,2 %<br>57,3 %<br>90,1 % | Bicyclo[3.3.1]non-2-en-9-on<br>Cyclooctancarbonsäureethylester<br>Cyclooctancarbonsäureethylester<br>Cyclooctandicarbonsäurediethylester |

## Ansprüche

1. Verfahren zur Herstellung von Cyclooctandicarbonsäurediestern durch Umsetzung von 1,5-Cyclooctadien mit Kohlenmonoxid und einem Alkohol,
dadurch gekennzeichnet,
daß man die Reaktion in Gegenwart einer Kobaltverbindung und eines tertiären Amins bei 100 bis 200 °C und 150 bis 350 bar durchführt.

2. Verfahren nach Anspruch 1,

dadurch gekennzeichnet,
daß man als Kobaltverbindung Salze, Oxide und/oder Komplexe des Kobalts einsetzt.

3. Verfahren nach Anspruch 1,
dadurch gekennzeichnet,
daß man als tertiäres Amin Pyridin und/oder nicht-ortho-substituierte Alkylpyridine verwendet.

4. Verfahren nach Anspruch 3,
dadurch gekennzeichnet,
daß man 4-Picolin verwendet.

5. Verfahren nach Anspruch 1,
dadurch gekennzeichnet,
daß man die Reaktion in einem Alkohol mit 1 bis 6 C-Atomen durchführt.

6. Verfahren nach Anspruch 1,
dadurch gekennzeichnet,
daß die Reaktion in einer ersten Stufe bei 120 bis 150 °C und in einer zweiten Stufe bei 150 bis 200 °C durchgeführt wird.

7. Verfahren nach Anspruch 1,
dadurch gekennzeichnet,
daß man die Reaktion bei einem Druck von 200 bis 300 bar durchführt.

8. Verfahren nach Anspruch 1,
dadurch gekennzeichnet,
daß man, bezogen auf eingesetztes 1,5-Cyclooctadien, 0,5 bis 5 Mol-% Kobaltverbindung verwendet.

9. Verfahren nach Anspruch 1,
dadurch gekennzeichnet,
daß das Molverhältnis tertiäres Amin : Kobaltverbindung 2 : 1 bis 10 : 1 beträgt.